(19) Europäisches Patentamt
European Patent Office
Office européen des brevets

(11) Numéro de publication : 0 317 846 B1

# (12) FASCICULE DE BREVET EUROPEEN

(45) Date de publication du fascicule du brevet : **16.10.91 Bulletin 91/42**

(51) Int. Cl.[5] : **A61K 31/62,** A61K 31/615

(21) Numéro de dépôt : **88118818.9**

(22) Date de dépôt : **11.11.88**

## (54) Compositions pharmaceutiques et cosmétiques à base de peroxyde de benzoyle et de salicylates lipophiles d'ammonium quaternaires et leur utilisation, notamment dans le traitement de l'acné.

Le dossier contient des informations techniques présentées postérieurement au dépôt de la demande et ne figurant pas dans le présent fascicule.

(30) Priorité : **24.11.87 FR 8716280**

(43) Date de publication de la demande : **31.05.89 Bulletin 89/22**

(45) Mention de la délivrance du brevet : **16.10.91 Bulletin 91/42**

(84) Etats contractants désignés : **AT BE CH DE FR GB IT LI NL SE**

(56) Documents cités :
**EP-A- 0 029 790**
**GB-A- 2 018 589**
**US-A- 4 514 385**

(73) Titulaire : **L'OREAL**
**14, Rue Royale**
**F-75008 Paris (FR)**

(72) Inventeur : **Philippe, Michel**
**3, Rue de l'Aubépine**
**F-92160 Antony (FR)**
Inventeur : **Hocquaux, Michel**
**70 Rue du Rendez vous**
**F75016 Paris (FR)**
Inventeur : **Sebag, Henri**
**26, Rue Erlanger**
**F-75016 Paris (FR)**
Inventeur : **Beck, Irina**
**70, Avenue G. Sachet**
**F-93240 Villepinte (FR)**
Inventeur : **Laugier, Jean-Pierre**
**22, Rue des Gouttières**
**F-92160 Antony (FR)**

(74) Mandataire : **Casalonga, Axel et al**
**BUREAU D.A. CASALONGA - JOSSE**
**Morassistrasse 8**
**W-8000 München 5 (DE)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des compositions pharmaceutiques et cosmétiques à base de peroxyde de benzoyle et de salicylates lipophiles d'ammonium quaternaires ainsi que leur utilisation, notamment dans le traitement de l'acné.

L'étiopathologie de l'acné bien que mal définie, prend son origine dans la formation d'une lésion caractéristique : le comédon. Celui-ci résulte de l'obstruction du canal pilosébacé par suite d'une dyskératinisation de la zone de l'infundibulum du canal. L'obstruction a pour effet majeur de modifier la rhéologie du sébum et les caractéristiques physicochimiques du milieu. Cette modification permet l'hyperprolifération des souches résidantes cutanées qui déclenche alors une réaction de type inflammatoire de l'organisme.

Parmi les agents thérapeutiques préconisés dans le traitement de l'acné, le peroxyde de benzoyle s'est avéré depuis déjà de nombreuses années être un agent kératolytique particulièrement intéressant et présentant en outre de bonnes propriétés bactériostatiques.

L'utilisation des antibiotiques classiques dans le traitement de l'acné est également très répandue. En effet, ils possèdent une activité bactériostatique et anti-inflammatoire importante. Les antibiotiques actifs par voie orale sont très nombreux, parmi ceux-ci, la clindamycine et surtout l'érythromycine présentent une activité par voie topique.

Pour augmenter l'activité des compositions antiacnéiques par voie topique, on a alors associé aux antibiotiques le peroxyde de benzoyle. En particulier, on a déjà associé l'érythromycine au peroxyde de benzoyle (brevet FR 7702157).

Cependant, les antibiotiques seuls ou en association avec le peroxyde de benzoyle présentent l'inconvénient majeur lors d'une utilisation prolongée, de rendre la flore bactérienne résistante et ils deviennent dès lors peu actifs lors de traitements ultérieurs. (LEYDEN J.J. J. Am. Acad. Dermatol. 8 (1) 41-45 (1983)).

En outre, cette association du peroxyde de benzoyle avec l'érythromycine s'avère instable dans le temps.

On a alors préconisé, (M. GLOOR Arch. Dermatol. Res. 265 207-212 (1979)) pour remplacer les antibiotiques, d'utiliser des ammonium quaternaires dans le traitement topique de l'acné. Il s'est avéré en effet que certains ammonium quaternaire étaient aussi actifs que les antibiotiques sur les principales souches responsables de l'acné, sans induire de phénomène de résistance.

Il est également déjà connu d'associer dans des compositions topiques le peroxyde de benzoyle aux ammonium quaternaires (brevet FR 7329233). Dans de telles compositions, le peroxyde de benzoyle agit en se décomposant et en libérant de l'oxygène actif in situ.

La demanderesse vient de découvrir de façon tout à fait surprenante qu'il était possible d'obtenir des compositions stables particulièrement efficaces dans le traitement de l'acné, mais aussi dans celui de l'ulcère cutané, des verrues et dyskératinisations de la peau et de manière générale dans le traitement de dermatoses et désordres cutanés, dans lesquelles le peroxyde de benzoyle, associé à certains dérivés ammonium quaternaires (les salicylates d'ammonium quaternaires) est stable, ne se décompose pas et reste actif.

La demanderesse a en effet constaté que lorsque le peroxyde de benzoyle est associé aux salicylates d'ammonium quaternaires, conformément à l'invention, dans des compositions topiques, sous forme de gel par exemple, le peroxyde de benzoyle au bout de deux mois de conservation ne s'est pas dégradé et est resté stable.

Du fait de la stabilité du peroxyde de benzoyle qui n'est pas dégradé lorsqu'il est associé aux salicylates d'ammonium quaternaires, conformes à l'invention, il est possible de l'employer à des doses moindres dans les compositions et d'augmenter ainsi la tolérance cutanée.

La demanderesse à également constaté que les salicylates d'ammonium quaternaires présentent un certain pouvoir absorbant des rayons ultraviolets et ainsi les compositions, conformes à l'invention, risquent peu d'induire les inconvénients dûs à l'utilisation du peroxyde de benzoyle : instabilité, effets secondaires...

Ces nouvelles compositions sont donc très stables et bien tolérées. Elles présentent de très bonnes propriétés antibactériennes sans induire de phénomène de résistance des souches, elles sont kératolytiques, bactériostatiques, notamment vis-à-vis de Propionibacterium Acnes qui est l'un des principaux germes responsables de l'acné, antiseptiques, bactéricides, antifongiques, et se montrent actives dans le traitement et la réduction du nombre des comédons.

Du fait de leurs propriétés, les compositions selon l'invention sont appropriées dans le traitement des désordres cutanés et de dermatoses, tels que notamment l'acné, l'ulcère cutané, les verrues et les dyskératinisations de la peau.

La présente invention a donc pour objet une composition topique pharmaceutique et cosmétique contenant du peroxyde de benzoyle et au moins un salicylate lipophile d'ammonium quaternaire dans un support physiologiquement acceptable.

Un autre objet de l'invention concerne l'utilisation des compositions pour la préparation d'un médicament

actif dans le traitement de l'acné.

L'invention a également pour objet une composition et un procédé de traitement cosmétique.

D'autres objets de l'invention apparaîtront à la lecture de la description et des exemples qui suivent.

Selon la présente invention, les salicylates lipophiles d'ammonium quaternaires associés au peroxyde de benzoyle, conformes à l'invention, sont des composés répondant essentiellement à la formule :

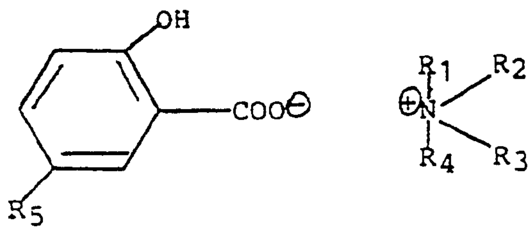 (I)

dans laquelle :

(i) $R_1$, $R_2$, $R_3$ et $R_4$, identiques ou différents, désignent un radical alkyle saturé, linéaire, en $C_1$-$C_{18}$, pouvant porter en bout de chaîne ou dans la chaîne, un ou plusieurs groupement(s) hydroxyle(s) ;

ou bien

(ii) $R_3$ et/ou $R_4$ désigne(nt) un groupement :

$$R_8-[OC_2H_3R_7]_n-[OCH_2-CHOH-CH_2]_p$$

dans lequel :

$$0 \leqq n \leqq 4 \text{ et p désigne 0 ou 1 ;}$$

$R_8$ désigne H ou un radical alkyle, alkényle, alkylcycloalkyle ou alkylaryle en $C_1$ à $C_{18}$, les groupements alkyles pouvant être linéaires ou ramifiés et les cycles aliphatiques ou aromatiques pouvant éventuellement porter un ou plusieurs substituant(s) alkyle ou alcoxy en $C_1$ à $C_4$ ;

$R_7$ désigne H, $CH_3$ ou $CH_2OH$.

Quand

$R_7$ désigne $CH_2OH$, $R_8$ est alors différent de H et p est égal à 1.

Dans les autres cas p = 0.

Le groupement ($OC_2H_3R_7$) peut désigner l'un et/ou l'autre des enchaînements suivants :

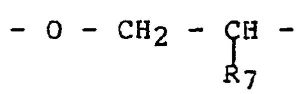 ; 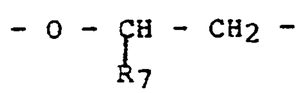

$R_1$ et $R_2$ ont les significations désignées au paragraphe (i) ;

(iii) $R_4$ désigne le groupement :

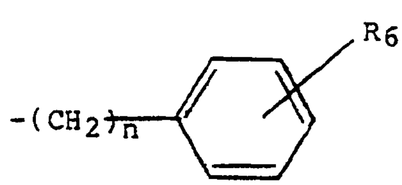

dans lequel n désigne 0 ou 1, auquel cas, $R_1$, $R_2$ et $R_3$ ont les significations indiquées ci-dessus, $R_6$ représente un hydrogène, un hydroxyle, un halogène, un reste alkyle ou hydroxyalkyle, ou un reste acyle en $C_1$ à $C_{18}$ ;

ou bien

(iv) $R_1$ et $R_2$ forment un hétérocycle aromatique, (auquel cas, $R_3$ n'existe pas) répondant à la formule :

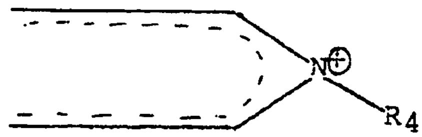

dans laquelle $R_4$ a les significations indiquées ci-dessus.
ou bien
(v) $R_1$ et $R_2$ forment un hétérocycle non aromatique, saturé ou insaturé, éventuellement interrompu par un atome d'oxygène ; auquel cas, $R_4$ représente un groupement défini ci-dessus et $R_3$ représente un groupement défini dans (i) ;
ou encore
(vi) $R_1$, $R_2$ et $R_3$ forment un bicycle non aromatique, saturé ou insaturé, $R_4$ représente dans ce cas un groupement défini ci-dessus.
Dans les différents cas évoqués ci-dessus, $R_5$ désigne un groupement :

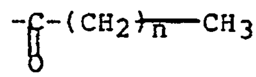

n variant de 0 à 16.

Ces composés sont décrits plus particulièrement dans la demande de brevet français n° 8616763 et sont préparés de préférence à partir d'un sel, tel que plus particulièrement, le carbonate des ammonium quaternaires correspondants, solubilisé en milieu alcoolique, et de préférence dans le méthanol, auquel on ajoute le dérivé d'acide salicylique lipophile choisi, solubilisé également dans un alcool tel que l'éthanol ou le méthanol, ou encore dans un éther comme le tétrahydrofuranne. La réaction démarre toute seule et est suivie d'un dégagement de gaz carbonique.

Des salicylates lipophiles d'ammonium quaternaires particulièrement préférés sont les suivants :
octanoyl-5 salicylate d'hexadécyl triméthylammonium,
décanoyl-5 salicylate d'hexadécyl triméthylammonium,
dodécanoyl-5 salicylate d'hexadécyl triméthylammonium,
octanoyl-5 salicylate d'hexadécyl pyridinium,
décanoyl-5 salicylate d'hexadécyl pyridinium,
dodécanoyl-5 salicylate d'hexadécyl pyridinium,
octanoyl-5 salicylate de benzyl diméthyl hexadécyl ammonium,
décanoyl-5 salicylate de benzyl diméthyl hexadécyl ammonium,
dodécanoyl-5 salicylate de benzyl diméthyl hexadécyl ammonium,
décanoyl-5 salicylate de benzyl triméthylammonium,
décanoyl-5 salicylate d'hexadécyl diméthyl hydroxyéthylammonium,
dodécanoyl-5 salicylate de tétraméthylammonium,
décanoyl-5 salicylate de dodécyl éthyl diméthylammonium,
décanoyl-5 salicylate de triméthyl-β-hydroxyéthyl ammonium,
dodécanoyl-5 salicylate de triméthyl-β-hydroxyéthyl ammonium,
décanoyl-5 salicylate de N-dodécyl N-méthylmorpholinium,
dodécanoyl-5 salicylate de N-méthyl N-octyl pipéridinium,
octanoyl-5 salicylate de benzéthonium,
dodécanoyl-5 salicylate de benzéthonium.

Ces composés peuvent être préparés comme indiqué ci-dessous.

A. Préparation d'octanoyl-5 salicylate de N-hexadécyl N,N,N-triméthylammonium.

A une solution de 2,62 g (4,16 mmoles) de carbonate d'hexadécyltriméthylammonium dissous dans 15 ml de méthanol, sont ajoutés 2 g (7,57 mmoles) d'acide octanoyl-5 salicylique dissous préalablement dans 15 ml de méthanol ; le mélange est agité 1 heure à température ambiante puis le solvant est évaporé et le résidu solide blanc obtenu est recristallisé dans un mélange acétone/ éther diéthylique pour conduire à 3,8 g (91,5% de rendement) d'octanoyl-5 salicylate d'hexadécyltriméthyl ammonium.

F = 130°C (acétone/éther diéthylique)

Analyse élémentaire : $C_{34}H_{61}NO_4$ ; M = 547,9

| | C | H | N |
|---|---|---|---|
| Calculé % | 74,54 | 11,22 | 2,56 |
| Trouvé % | 74,48 | 11,31 | 2,62 |

Les spectres de RMN $^1H$ et $^{13}C$ confirment la structure attendue avec les valeurs caractéristiques du cation ammonium et de l'anion octanoyl-5 salicylate.

B. Préparation du décanoyl-5 salicylate de N-hexadécyl N,N,N-triméthylammonium.

A une solution de 2,2 g (3,5 mmoles) de carbonate d'hexadécyltriméthylammonium dissous dans 15 ml de méthanol, sont ajoutés 2 g (6,85 mmoles) d'acide décanoyl-5 salicylique dissous préalablement dans 15 ml d'éthanol ; le mélange est agité 1 heure à température ambiante, plus les solvants sont évaporés et le résidu solide blanc obtenu est recristallisé dans un mélange acétone/éther diéthylique pour conduire à 3,8 g (96% de rendement) de décanoyl-5 salicylate d'hexadécyltriméthyl ammonium.

F = 130°C (acétone/éther diéthylique)

Analyse élémentaire : $C_{36}H_{65}NO_4$ ; M = 575,9

| | C | H | N |
|---|---|---|---|
| Calculé % | 75,08 | 11,37 | 2,43 |
| Trouvé % | 75,05 | 11,42 | 2,44 |

Les spectres de RMN $^1H$ et $^{13}C$ confirment la structure attendue avec les valeurs caractéristiques du cation ammonium quaternaire et de l'anion décanoyl-5 salicylate.

C. Préparation du dodécanoyl-5 salicylate de N-hexadécyl N,N,N-triméthylammonium.

A une solution de 2 g (3,18 mmoles) de carbonate d'hexadécyltriméthylammonium dissous dans 15 ml de méthanol, sont ajoutés 2 g (6,25 mmoles) d'acide dodécanoyl-5 salicylique dissous préalablement dans 15 ml d'éthanol ; le mélange est agité 1 heure à température ambiante, puis les solvants sont évaporés et le résidu solide blanc obtenu est recristallisé dans un mélange acétone/éther diéthylique pour conduire à 3,5 g (93% de rendement) de dodécanoyl-5 salicylate d'hexadécyltriméthylammonium.

F = 130°C (acétone/éther diéthylique)

Analyse élémentaire : $C_{38}H_{69}NO_4$ ; M = 604

| | C | H | N |
|---|---|---|---|
| Calculé % | 75,57 | 11,52 | 2,32 |
| Trouvé % | 75,17 | 11,61 | 2,5 |

Le spectre de RMN de $^{13}C$ confirme la structure attendue avec les valeurs caractéristiques du cation ammonium quaternaire et de l'anion dodécanoyl-5 salicylate.

Le spectre UV (éthanol) démontre une absorption $\lambda_{max}$ = 280 nm ; le coefficient d'extinction molaire ε = 14080.

D. Préparation de l'octanoyl-5 salicylate de N-hexadécylpyridinium-monohydrate.

A une solution de 2,5 g (3,79 mmoles) de carbonate d'hexadécylpyridinium dissous dans 15 ml de méthanol, sont ajoutés 2 g (7,57 mmoles) d'acide octanoyl-5 salicylique dissous préalablement dans 15 ml de méthanol ; le mélange est agité 1 heure à température ambiante, puis le résidu brut marron obtenu est filtré et le filtrat est évaporé à sec pour conduire à 4,2 g (98% de rendement) d'octanoyl-5 salicylate d'hexadécylpyridinium.

Analyse élémentaire : $C_{36}H_{57}NO_4, 1H_2O$ ; M = 585,9

| | C | H | N |
|---|---|---|---|
| Calculé % | 73,8 | 10,15 | 2,39 |
| Trouvé % | 73,22 | 9,77 | 2,31 |

Le spectre de RMN du $^{13}C$ confirme la structure attendue avec les valeurs caractéristiques du cation ammonium quaternaire et de l'anion octanoyl-5 salicylate.

La spectre UV (éthanol) démontre une absorption $\lambda_{max}$ = 279 nm ; le coefficient d'extinction molaire ε = 21450.

E. Préparation du décanoyl-5 salicylate de N-hexadécylpyridinium-sesquihydrate.

A une solution de 2,3 g (3,44 mmoles) de carbonate d'hexadécylpyridinium dissous dans 15 ml de méthanol, sont ajoutés 2 g (6,85 mmoles) d'acide décanoyl-5 salicylique dissous préalablement dans 15 ml d'éthanol; le mélange est agité 1 heure à température ambiante, puis les solvants sont évaporés et le résidu marron obtenu est recristallisé dans un mélange acétone/éther diéthylique pour conduire à 3,9 g (96% de rendement) de décanoyl-5 salicylate d'hexadécylpyridinium.

F = 73°C (acétone/éther diéthylique)

Analyse élémentaire : $C_{38}H_{61}NO_4, 1,5H_2O$ ; M = 622,9

| | C | H | N |
|---|---|---|---|
| Calculé % | 73,27 | 10,36 | 2,25 |
| Trouvé % | 73,64 | 9,64 | 1,82 |

La spectre de RMN du $^{13}C$ confirme la structure attendu avec les valeurs caractéristiques du cation ammonium quaternaire aromatique et du décanoyl-5 salicylate.

F. Préparation du dodécanoyl-5 salicylate de N-hexadécylpyridinium-hydrate.

A une solution de 2,1 g (3,14 mmoles) de carbonate d'hexadécylpyridinium dissous dans 15 ml de méthanol, sont ajoutés 2 g (6,25 mmoles) d'acide dodécanoyl-5 salicylique dissous préalablement dans 15 ml d'éthanol ; le mélange est agité 1 heure à température ambiante, puis les solvants sont évaporés et le résidu marron obtenu est recristallisé dans un mélange acétone/éther diéthylique pour conduire à 3,7 g (95% de rendement) de dodécanoyl-5 salicylate d'hexadécylpyridinium.

F = 79°C (acétone/éther diéthylique)

Analyse élémentaire : $C_{40}H_{65}NO_4$, $1H_2O$ ; M = 642

| | C | H | N |
|---|---|---|---|
| Calculé % | 74,84 | 10,52 | 2,18 |
| Trouvé % | 74,61 | 10,12 | 1,96 |

Le spectre de RMN du $^{13}C$ confirme la structure attendue avec les valeurs caractéristiques du cation ammonium quaternaire aromatique et du dodécanoyl-5 salicylate.

Le spectre UV (éthanol) démontre une absorption $\lambda_{max}$ = 279 nm ; le coefficient d'extinction molaire ε = 18910.

G. Préparation de l'octanoyl-5 salicylate de N-benzyl N,N-diméthyl N-hexadécylammonium.

A une solution de 3 g (3,84 mmoles) de carbonate de benzyldiméthyl hexadécylammonium dissous dans 15 ml de méthanol, sont ajoutés 2 g (7,57 mmoles) d'acide octanoyl-5 salicylique dissous préalablement dans 15 ml de méthanol ; le mélange est agité 1 heure à température ambiante, puis le solvant est évaporé et le résidu blanc est recristallisé dans un mélange acétone/éther diéthylique pour conduire à 4,5 g (95% de rendement) d'octanoyl-5 salicylate de benzyldiméthylhexadécylammonium.

F = 114°C (acétone/éther diéthylique)

Analyse élémentaire : $C_{40}H_{65}NO_4$ ; M = 623,9

| | C | H | N |
|---|---|---|---|
| Calculé % | 77,00 | 10,50 | 2,24 |
| Trouvé % | 77,02 | 10,58 | 2,38 |

Le spectre de RMN du $^{13}C$ confirme la structure attendue avec les signaux caractéristiques du cation ammonium quaternaire et de l'octanoyl-5 salicylate.

Les composés H à Q sont préparés d'une manière analogue à celle décrite dans les exemples A à G.

H. Préparation du décanoyl-5 salicylate de N-benzyl N,N-diméthyl N-hexadécylammonium.

F = 113°C (acétone/éther diéthylique)

Analyse élémentaire : $C_{42}H_{69}NO_4$ ; M = 652

| | C | H | N |
|---|---|---|---|
| Calculé % | 77,37 | 10,67 | 2,15 |
| Trouvé % | 77,22 | 10,69 | 2,25 |

I. Préparation du dodécanoyl-5 salicylate de N-benzyl N,N-diméthyl N-hexadécylammonium.

F = 117°C (acétone/éther diéthylique)

Analyse élémentaire : $C_{44}H_{73}NO_4$ ; M = 680,08

| | C | H | N |
|---|---|---|---|
| Calculé % | 77,71 | 10,82 | 2,06 |
| Trouvé % | 77,15 | 10,81 | 2,08 |

Le spectre UV (éthanol) démontre une absorption $\lambda_{max}$ = 281 nm ; le coefficient d'extinction molaire ε = 17720.

J. Préparation du décanoyl-5 salicylate de N-benzyl N,N,N-triméthylammonium.

F = 135°C (acétone/éther diéthylique)

Analyse élémentaire : $C_{27}H_{39}NO_4$ ; M = 441,6

| | C | H | N |
|---|---|---|---|
| Calculé % | 73,43 | 8,9 | 3,17 |
| Trouvé % | 73,01 | 8,74 | 3,18 |

K. Préparation du décanoyl-5 salicylate de N-hexadécyl N,N-diméthyl N,β-hydroxyéthylammonium.

Analyse élémentaire : $C_{37}H_{68}NO_5$, $1/2H_2O$ ; M = 615,96

| | C | H | N |
|---|---|---|---|
| Calculé % | 72,15 | 11,29 | 2,12 |
| Trouvé % | 72,51 | 10,85 | 2,27 |

L. Préparation du dodécanoyl-5 salicylate de N,N, N,N-tétraméthylammonium.

F = 97°C (acétone/éther diéthylique)

Analyse élémentaire : $C_{23}H_{40}NO_4$ ; M = 390,15

| | C | H | N |
|---|---|---|---|
| Calculé % | 70,8 | 10,33 | 3,59 |
| Trouvé % | 70,27 | 9,87 | 3,38 |

M. Préparation du dodécanoyl-5 salicylate de triméthyl β-hydroxyéthylammonium.

F = 67°C (méthanol/éther diéthylique)

Analyse élémentaire : $C_{24}H_{41}NO_5$ ; M = 423,6

| | C | H | N |
|---|---|---|---|
| Calculé % | 68,05 | 9,76 | 3,31 |
| Trouvé % | 68,09 | 9,59 | 2,69 |

Spectre UV (éthanol) = $\lambda_{max}$=280 nm; $\varepsilon$=16640

N. Préparation du décanoyl-5 salicylate de triméthyl β-hydroxyéthylammonium.

Analyse élémentaire : $C_{22}H_{35}NO_5 3/4H_2O$ ; M = 407,6

| | C | H | N |
|---|---|---|---|
| Calculé % | 64,82 | 9,51 | 3,43 |
| Trouvé % | 64,75 | 9,36 | 3,01 |

O. Préparation du décanoyl-5 salicylate de diméthyl éthyl dodécyl ammonium.

F = 65°C (acétone/éther diéthylique)

Analyse élémentaire : $C_{33}H_{59}NO_4,H_2O$; M = 551,9

| | C | H | N |
|---|---|---|---|
| Calculé % | 71,8 | 11,14 | 2,62 |
| Trouvé % | 72,07 | 10,74 | 2,33 |

P. Préparation du dodécanoyl-5 salicylate de N-méthyl, N-octyl pipéridinium.

F= 45°C (acétone/éther diéthylique)

Analyse élémentaire : $C_{33}H_{57}NO_4$,0,5 $H_2O$; M = 540,9

| | C | H | N |
|---|---|---|---|
| Calculé % | 73,28 | 10,81 | 2,59 |
| Trouvé % | 73,31 | 11,02 | 2,7 |

Q. Préparation du décanoyl-5 salicylate de N-dodécyl, N-méthyl morpholinium.

F = 78°C (acétone/éther diéthylique)

Analyse élémentaire : $C_{34}H_{59}NO_5$ ; M = 561,8

| | C | H | N |
|---|---|---|---|
| Calculé % | 72,68 | 10,58 | 2,49 |
| Trouvé % | 72,58 | 10,68 | 2,6 |

R. Préparation de l'octanoyl-5 salicylate de benzéthonium

A une solution de 3,7 g (4,2 mmoles) de carbonate de benzéthonium dissous dans 100 ml de méthanol, sont ajoutés 2,2 g (8,4 mmoles) d'acide octanoyl-5 salicylate dissous préalablement dans 100 ml de méthanol; le mélange est agité 1 heure à température ambiante puis la solution est filtrée et le filtrat est évaporé à sec pour conduire à 5,5 g (98% de rendement) d'octanoyl-5 salicylate de benzéthonium.

Analyse élémentaire : $C_{42}H_{61}NO_6,2H_2O$;PM=712

| | C | H | N |
|---|---|---|---|
| Calculé % | 70,85 | 9,20 | 1,97 |
| Trouvé % | 70,93 | 9,07 | 2,07 |

Le spectre de RMN du $^{13}C$ confirme la structure attendue avec les valeurs caractéristiques du cation ammonium quaternaire et de l'anion octanoyl-5 salicylate.

S. Préparation du dodécanoyl-5 salicylate de benzéthonium

A une solution de 2,82 g (3,2 mmoles) de carbonate de benzéthonium dissous dans 100 ml de méthanol, sont ajoutés 2 g (6,4 mmoles) d'acide dodécanoyl-5 salicylique dissous préalablement dans 100 ml d'éthanol; le mélange est agité 1 heure à température ambiante, puis la solution est filtrée et le filtrat est évaporé à sec pour conduire à 4,5 g (97% de rendement) de dodécanoyl-5 salicylate de benzéthonium.

Analyse élémentaire : $C_{46}H_{69}NO_6,H_2O$;PM=750

| | C | H | N |
|---|---|---|---|
| Calculé % | 73,66 | 9,54 | 1,87 |
| Trouvé % | 73,60 | 9,48 | 1,85 |

Le spectre de RMN du $^{13}C$ confirme la structure attendue avec les valeurs caractéristiques du cation ammonium quaternaire et de l'anion dodécanoyl-5 salicylate.

Les compositions pharmaceutiques ou cosmétiques, objet de l'invention, sont essentiellement caractérisées par le fait qu'il s'agit de compositions topiques, contenant en association, dans un support physiologiquement acceptable, du peroxyde de benzoyle et au moins un salicylate lipophile d'ammonium quaternaire, répondant à la formule générale (I) ci-dessus.

Les compositions peuvent se présenter sous forme de solutions, d'émulsions, de suspensions, de gels ou de dispersions, contenant au moins un composé répondant à la formule (I) dans des concentrations comprises entre 0,01 et 25% en poids, par rapport au poids total de la composition et de préférence comprises entre 0,1 et 3% en poids et du peroxyde de benzoyle dans des concentrations comprises entre 0,1 et 20% en poids par rapport au poids total de la composition et de préférence comprises entre 1 et 10% en poids.

Ces compositions peuvent contenir des véhicules et adjuvants physiologiquement acceptables, bien connus dans l'état de la technique. On peut, par exemple, préparer les solutions, micro-suspensions ou émulsions vésiculaires, en utilisant un ou plusieurs solvant(s) organique(s) acceptable(s) du point de vue physiologique, choisi(s) en plus de l'eau parmi les solvants tels que l'acétone, l'éthanol, l'alcool isopropylique, les éthers de glycol tels que les produits vendus sous la dénomination de "DOWANOL"®, les polyglycols, les polyéthylèneglycols, les esters d'alkyle en $C_1$-$C_4$ d'acide à courte chaîne, de préférence les lactates d'éthyle ou d'isopropyle, les triglycérides d'acides gras tels que les produits commercialisés sous la dénomination de "MIGLYOL"® et le myristate d'isopropyle.

Les compositions conformes à l'invention peuvent également renfermer des agents épaississants et gélifiants choisis par exemple parmi la cellulose et ses dérivés, la gomme de guar, les hétérobiopolysaccharides, l'acide polyacrylique réticulé, le copolymère d'acide methacrylique/methacrylate de méthyle, la poly-β-alanine, la silice colloïdale, des adoucissants, des surgraissants, des émollients, des mouillants, des agents de surface, des régulateurs de pH, des agents de pénétration, des conservateurs, des agents antimousses, des filtres solaires, des huiles, des cires, des parfums, des colorants et/ou des pigments ayant pour fonction de colorer la peau ou la composition elle-même et tout autre ingrédient habituellement utilisé dans des compositions destinées à une application topique.

Bien entendu, les excipients et ingrédients qui pourraient réagir de façon indésirable avec le peroxyde de benzoyle, utilisé conformément à l'invention, doivent être exclus.

Les compositions conformes à l'invention peuvent également contenir en association des agents antiacnéiques tels que les dérivés rétinoïques, les agents antibactériens, les anti-inflammatoires, les stéroïdiens à action non hormonale, notamment la pregnénolone, et/ou des agents kératolytiques ou comédolytiques.

Les formes galéniques principalement conditionnées pour la voie topique se présentent notamment sous forme de crèmes, de laits, de gels, de dispersions ou micro-émulsions, de compositions plus ou moins épaissies, de tampons imbibés, de pommades, de sticks ou sous forme de pains de savon.

Les compositions pharmaceutiques qui sont un objet de l'invention sont caractérisées par le fait qu'elles contiennent le peroxyde de benzoyle et au moins un salicylate lipophile d'ammonium quaternaire de formule (I) dans un support pharmaceutiquement acceptable.

Les compositions pharmaceutiques, conformes à l'invention, du fait de leurs propriétés antibactériennes et kératolytiques notamment, peuvent être utilisées à titre de médicament dans le traitement thérapeutique de dermatoses, et en particulier de l'acné.

Un autre objet de l'invention est donc également constitué par l'utilisation des compositions pharmaceutiques pour la préparation d'un médicament destiné au traitement de dermatoses, tels qu'en particulier l'acné, les ulcères cutanés, les verrues et dyskératinisations de la peau.

Le traitement thérapeutique de l'acné consiste à appliquer sur les zones acnéiques la composition définie ci-dessus en deux ou trois applications journalières pendant 3 à 25 semaines suivant la gravité du cas considéré.

Les compositions cosmétiques qui sont un autre objet de l'invention sont caractérisés par le fait qu'elles contiennent le peroxyde de benzoyle et au moins un salicylate lipophile d'ammonium quaternaire de formule (I) dans un support cosmétiquement acceptable.

Ces compositions cosmétiques, conformes à l'invention, peuvent être utilisées pour le traitement cosmétique de la peau, notamment comme produit comédolytique, kératolytique ou verrulytique.

Un autre objet de l'invention concerne un procédé de traitement cosmétique caractérisé par le fait qu'il consiste à appliquer sur la peau une composition conforme à l'invention en vue d'assainir ou d'épurer cette dernière.

Les exemples ci-après sont destinés à illustrer l'invention sans pour autant présenter un caractère limitatif.

On prépare les compositions suivantes :

## EXEMPLE 1

| | | |
|---|---|---|
| - Peroxyde de benzoyle 100% de matière active | | 5,0 g |
| - Octanoyl-5 salicylate d'hexadécyltriméthylammonium | | 1,0 g |
| - Diéthylèneglycol monoéthyléther | | 20,0 g |
| - Ethanol 90° | | 50,0 g |
| - Hydroxyéthyl cellulose | | 2,0 g |
| - Eau purifiée | qsp | 100,0 g |

Cette composition se présente sous forme de gel.

## EXEMPLE 2

| | | |
|---|---|---|
| - Peroxyde de benzoyle à 100% de matière active | | 10,0 g |
| - Dodécanoyl-5 salicylate d'hexadécyltriméthylammonium | | 0,5 g |
| - Propylèneglycol | | 10,0 g |
| - Ethanol | | 40,0 g |
| - Hydroxyéthyl cellulose | | 2,2 g |
| - Silice colloïdale | | 2,0 g |
| - Propylèneglycol monoéthyléther | | 15,0 g |
| - Eau purifiée | qsp | 100,0 g |

Cette compositions se présente sous forme de gel.

## EXEMPLE 3

| | | |
|---|---|---|
| - Peroxyle de benzoyle à 100% de matière active | | 5,0 g |
| - Décanoyl-5 salicylate d'hexadécyl-triméthylammonium | | 1,0 g |
| - Stéarate de polyéthylèneglycol oxyéthylé à 50 moles d'oxyde d'éthylène | | 4,2 g |
| - Monostéarate de glycérol | | 1,1 g |
| - Alcool cétylique | | 2,0 g |
| - Alcool stéarylique | | 2,2 g |
| - Octyl dodécanol | | 17,0 g |
| - Hydroxyéthyl cellulose | | 0,35 g |
| - Eau purifiée | qsp | 100,0 g |

Cette composition se présente sous forme de crème.

## EXEMPLE 4

| | | |
|---|---|---|
| - Peroxyde de benzoyle à 100% de matière active | | 10,0 g |
| - Dodécanoyl-5 salicylate d'hexadécyl-triméthylammonium | | 0,5 g |
| - Stéarate de PEG 75 (polyéthylèneglycol oxyéthylé à 75 moles d'oxyde d'éthylène + monostéarate de glycol, vendu par la Société GATTEFOSSE sous la dénomination commerciale de GELOT® 64) | | 14,0 g |
| - MIGLYOL 812® (triglycérides d'acides gras en $C_6$-$C_{12}$, vendu par la Société DYNAMIT NOBEL) | | 17,0 g |
| - Alcool cétylique | | 2,0 g |
| - Alcool stéarylique | | 2,0 g |
| - Hydroxypropyl guar | | 0,3 g |
| - Eau purifiée | qsp | 100,0 g |

Cette composition se présente sous forme de gel.

## EXEMPLE 5

| | | | |
|---|---|---|---|
| - Peroxyde de benzoyle 100% de matière active | | 2,5 | g |
| - Octanoyl-5 salicylate d'hexadécyl-triméthylammonium | | 1,0 | g |
| - Diéthylèneglycol monoéthyléther | | 20,0 | g |
| - Ethanol 90° | | 50,0 | g |
| - Hydroxyéthyl cellulose | | 2,0 | g |
| - Eau purifiée | qsp | 100,0 | g |

Cette composition se présente sous forme de gel.

Lorsque les compositions selon les exemples 1 à 5 sont appliquées quotidiennement sur des peaux à tendance acnéïques ; on constate au bout de 3 à 6 semaines du assainissement de ces dernières qui deviennent notamment moins grasses et une diminution du nombre de comédons.

## Revendications

1. Compositions pharmaceutique ou cosmétique, caractérisée par le fait qu'il s'agit d'une composition topique contenant en association dans un support physiologiquement acceptable, du peroxyde de benzoyle et au moins un salicylate lipophile d'ammonium quaternaire répondant à la formule générale (I) :

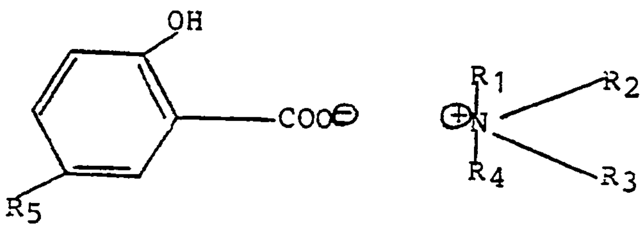

(I)

dans laquelle :

(i) $R_1$, $R_2$, $R_3$ et $R_4$, identiques ou différents, désignent un radical alkyle saturé, linéaire en $C_1$-$C_{18}$, pouvant porter en bout de chaîne ou dans la chaîne un ou plusieurs groupement(s) hydroxyle(s) ;

(ii) $R_3$ et/ou $R_4$ désigne(nt) un groupement :

$$R_8-\left[OC_2H_3R_7\right]_n-\left[OCH_2-CHOH-CH_2\right]_p-$$

dans lequel :

$$0 \leqq n \leqq 4 \text{ et p désigne 0 ou 1 ;}$$

$R_8$ désigne H ou un radical alkyle, alkényle, alkylcycloalkyle ou alkylaryle en $C_1$ à $C_{18}$, les groupements alkyles pouvant être linéaires ou ramifiés et les cycles aliphatiques ou aromatiques pouvant éventuellement porter un ou plusieurs substituant(s) alkyle ou alcoxy en $C_1$ à $C_4$ ;

$R_7$ désigne H, $CH_3$ ou $CH_2OH$ ;

Quand

$R_7$ désigne $CH_2OH$, $R_8$ est alors différent de H et p est égal à 1 ;
Dans les autres cas p = 0 ;
Le groupement ($OC_2H_3R_7$) peut désigner l'un et/ou l'autre des enchaînements suivants :

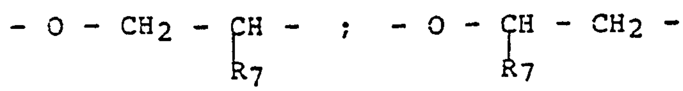

$R_1$ et $R_2$ ont les significations désignées au paragraphe (i) ;
(iii) $R_4$ désigne le groupement :

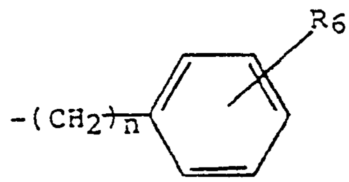

dans lequel n désigne 0 ou 1, $R_1$, $R_2$, et $R_3$ ayant les mêmes significations que sous (i) ; $R_6$ représente un hydrogène, un hydroxyle, un halogène, un reste alkyle ou hydroxyalkyle ou un reste acyle en $C_1$ à $C_{18}$ ;
(iv) $R_1$ et $R_2$ peuvent former un hétérocycle aromatique (auquel cas $R_3$ n'existe pas) répondant à la formule:

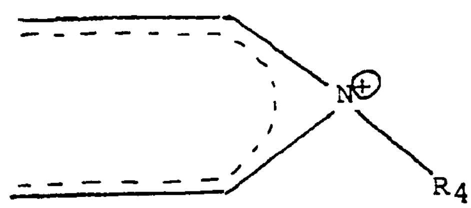

dans laquelle $R_4$ a les significations indiquées ci-dessus ; ou
(v) $R_1$ et $R_2$ désignent un hétérocycle non aromatique, saturé ou insaturé, éventuellement interrompu par un atome d'oxygène ; $R_4$ représente un groupement défini ci-dessus et $R_3$ représente un groupement défini dans le paragraphe (i) ;
(vi) $R_1$, $R_2$ et $R_3$ peuvent former un bicycle non aromatique, saturé ou insaturé ; $R_4$ représente un groupement défini ci-dessus ; et
$R_5$ désigne un groupement répondant à la formule :

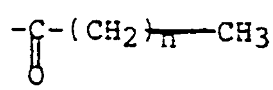

dans laquelle n est un nombre entier variant entre 0 à 16.

2. Composition selon la revendication 1, caractérisée par le fait que dans la formule (I) du salicylate lipophile d'ammonium quaternaire, $R_4$ désigne un groupement benzyle et $R_1$, $R_2$, $R_8$ ont les significations indiquées sous (i).

3. Composition selon l'une quelconque des revendications 1 ou 2, caractérisée par le fait que le salicylate lipophile d'ammonium quaternaire est choisi parmi les composés suivants : octanoyl-5 salicylate d'hexadécyl triméthylammonium, décanoyl-5 salicylate d'hexadécyl triméthylammonium, dodécanoyl-5 salicylate d'hexadécyl triméthylammonium, octanoyl-5 salicylate d'hexadécyl pyridinium, décanoyl-5 salicylate d'hexadécyl pyridinium, dodécanoyl-5 salicylate d'hexadécyl pyridinium, octanoyl-5 salicylate de benzyl diméthyl hexadécyl ammonium, décanoyl-5 salicylate de benzyl diméthyl hexadécyl ammoniumn, dodécanoyl-5 salicylate de benzyl diméthyl hexadécyl ammonium, décanoyl-5 salicylate de benzyl triméthylammonium, décanoyl-5 salicylate d'hexadécyl diméthyl hydroxyéthyl ammonium, dodécanoyl-5 salicylate de tétraméthyl ammonium, décanoyl-5 salicylate de dodécyl éthyl diméthylammonium, décanoyl-5 salicylate de triméthyl-β-hydroxyéthyl ammonium,

dodécanoyl-5 salicylate de triméthyl-β-hydroxyéthylammonium, décanoyl-5 salicylate de N-dodécyl N-méthylmorpholinium, dodécanoyl-5 salicylate de N-méthyl N-octyl pipéridinium, octanoyl-5 salicylate de benzéthonium, dodécanoyl-5 salicylate de benzéthonium.

4. Composition selon l'une quelconque des revendications 1 à 3, caractérisée par le fait que le peroxyde de benzoyle est présent dans des proportions de 0,1 à 20% en poids par rapport au poids total de la composition.

5. Composition selon l'une quelconque des revendications 1 à 4, caractérisée par le fait que le salicylate lipophile d'ammonium quaternaire est présent dans les proportions de 0,01 à 25% en poids, par rapport au poids total de la composition.

6. Composition selon la revendication 4, caractérisée par le fait que le peroxyde de benzoyle est présent dans des proportions de 1 à 10% en poids par rapport au poids total de la composition.

7. Composition selon la revendication 5, caractérisée par le fait que le salicylate lipophile d'ammonium quaternaire est présent dans des proportions de 0,1 à 3% en poids par rapport au poids total de la composition.

8. Composition selon l'une quelconque des revendications 1 à 7, pour son application à titre de médicament, caractérisée par le fait que le support approprié est un support pharmaceutiquement acceptable.

9. Composition selon l'une quelconque des revendications 1 à 7, caractérisée par le fait que le support approprié est un support cosmétiquement acceptable.

10. Composition selon l'une quelconque des revendications 1 à 9, caractérisée par le fait qu'elle se présente sous forme de gels, de solutions, de dispersions, d'émulsions ou de suspensions.

11. Compositions selon l'une quelconque des revendications 1 à 10, caractérisée par le fait qu'elle se présente sous forme de crème, de lait, de gel, de dispersion ou micro-émulsion, de compositions plus ou moins épaissie, de tampon imbibé, de pommade, de stick ou sous forme de pain de savon.

12. Compositions selon l'une quelconque des revendications 1 à 11, caractérisée par le fait qu'elle comprend en outre au moins un autre agent antiacnéique.

13. Composition selon l'une quelconque des revendications 1 à 12, caractérisée par le fait qu'elle comprend en outre au moins un filtre solaire.

14. Utilisation des compositions définies selon la revendication 8, pour la préparation d'un médicament pour le traitement de dermatoses.

15. Utilisation des compositions selon la revendication 14, pour le traitement des ulcères cutanés, des verrues et dyskératinisations de la peau.

16. Utilisation des compositions définies selon la revendication 8 pour la préparation d'un médicament pour le traitement de l'acné.

17. Procédé de traitement cosmétique, caractérisé par le fait qu'il consiste à appliquer sur la peau une composition telle que définie dans la revendication 9, en vue d'assainir ou d'épurer la peau.

**Patentansprüche**

1. Pharmazeutische oder kosmetische Zusammensetzung, dadurch **gekennzeichnet**, das es sich um eine topische Zusammensetzung handelt, die zusammen mit einem physiologisch akzeptablen Träger Benzoylperoxid und zumindest ein lipophiles quartäres Ammoniumsalicylat der folgenden allgemeinen Formel (I) umfasst:

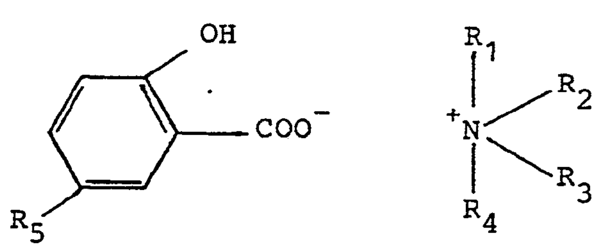

(I)

worin bedeuten :

(i) $R_1$, $R_2$, $R_3$ und $R_4$, die identisch oder veschieden sein können, bezeichnen ein gesättigtes lineares $C_{1-18}$ -Alkylradikal, welches am Ende der Kette oder innerhalb der Kette eine oder mehrere Hydroxylgruppen aufweisen kann ;

(ii) $R_3$ und/oder $R_4$ bedeuten die Gruppierung :

$$R_8-[OC_2H_3R_7]_n-[OCH_2-CHOH-CH_2]_p-$$

worin $0 \leq n \leq 4$ und worin p 0 oder 1 bedeutet; worin $R_8$ H oder ein Alkylradikal, Alkenylradikal, Alkylcycloalkylradikal oder Alkylarylradikal mit 1 bis 18 C-Atomen bedeutet, wobei die Alkylgruppen linear oder verzweigt sein können und wobei die aliphatischen oder aromatischen Zyklen gegebenenfalls einen oder mehrere Alkylsubstituenten oder Alkoxysubstituenten mit 1 bis 4 C-Atomen aufweisen können,
worin $R_7$ H, $CH_3$ oder $CH_2OH$ bedeutet;
wenn $R_7$ $CH_2OH$ ist, weist $R_8$ eine andere Bedeutung als H auf und ist p gleich 1;
in den anderen Fällen ist p = 0;
die Gruppe ($OC_2H_3R_7$) kann die eine und/oder die andere der folgenden Verknüpfungen aufweisen:

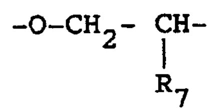 ; 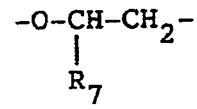

$R_1$ und $R_2$ haben die in Abschnitt (i) angegebenen Bedeutungen;
(iii) $R_4$ bedeutet die Gruppe

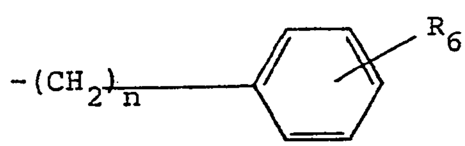

worin n 0 oder 1 bedeutet, worin $R_1$, $R_2$ und $R_3$ die gleichen Bedeutungen wie oben unter (i) aufweisen; $R_6$ bedeutet einen Wasserstoff-, einen Hydroxyl-, einen Halogen-, einen Alkyl- oder Hydroxyalkyl- oder einen Acylrest mit 1 bis 18 C-Atomen;
(iv) $R_1$ und $R_2$ können einen aromatischen Heterozyklus der Formel aufweisen (in diesem Fall ist $R_3$ nicht mehr vorhanden)

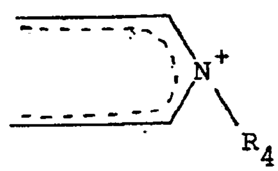

worin $R_4$ die oben angegebenen Bedeutungen aufweist; oder
(v) $R_1$ und $R_2$ bedeuten einen nicht-aromatischen Heterozyklus, gesättigt oder ungesättigt, der gegebenenfalls durch ein Sauerstoffatom unterbrochen sein kann; $R_4$ bedeutet eine oben definierte Gruppe und $R_3$ bedeutet eine in Abschnitt (i) definierte Gruppe;
(vi) $R_1$, $R_2$ und $R_3$ können einen nicht-aromatischen Bizyklus, gesättigt oder ungesättigt, bilden; $R_4$ bedeutet eine oben definierte Gruppe; und
$R_5$ bedeutet eine Gruppe der Formel

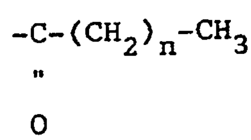

worin n eine ganze Zahl zwischen 0 und 16 bedeutet.

2. Zusammensetzung nach Anspruch 1, dadurch **gekennzeichnet**, dass in der Formel (I) des lipophilen quartären Ammoniumsalicylates $R_4$ eine Benzylgruppe und $R_1$, $R_2$ und $R_3$ die unter (i) angegebenen Bedeutungen aufweisen.

3. Zusammensetzung nach einem der Ansprüche 1 oder 2, dadurch **gekennzeichnet**, dass das lipophile quartäre Ammoniumsalicylat aus den folgenden Verbindungen ausgewählt ist : Hexadecyltrimethylammonium-5-octanoyl-salicylat, Hexadecyltrimethylammonium-5-decanoyl-salicylat, Hexadecyltrimethylammonium-5-dodecanoyl-salicylat, Hexadecylpyridinium-5-octanoyl-salicylat, Hexadecylpyridinium-5-decanoyl-salicylat, Hexadecylpyridinium-5-dodecanoyl-salicylat, Benzyldimethylhexadecylammonium-5-octanoyl-salicylat, Benzyldimethylhexadecylammonium-5-decanoyl-salicylat, Benzyldimethylhexadecylammonium-5-dodecanoyl-salicylat, Benzyltrimethylammonium-5-decanoyl-salicylat, Hexadecyldimethylhydroxyethylammonium-5-decanoyl-salicylat, Tetramethylammonium-5-dodecanoyl-salicylat, Dodecylethyldimethylammonium-5-decanoyl-salicylat, Trimethyl-beta-hydroxyethylammonium-5-decanoyl-salicylat, Trimethyl-beta-hydroxyethylammonium-5-dodecanoyl-salicylat, N-Dodecyl-N-methylmorpholinium-5-decanoyl-salicylat, N-Methyl-N-octylpiperidinium-5-dodecanoyl-salicylat, Benzethonium-5-octanoyl-salicylat, Benzethonium-5-dodecanoyl-salicylat.

4. Zusammensetzung nach einem der Ansprüche 1 bis 3, dadurch **gekennzeichnet**, dass das Benzoylperoxid in einer Menge von 0,1 bis 20 Gew.%, bezogen auf das Gesamtgewicht der Zusammensetzung, vorhanden ist.

5. Zusammensetzung nach einem der Ansprüche 1 bis 4, dadurch **gekennzeichnet**, dass das lipophile quartäre Ammoniumsalicylat in einer Menge von 0,01 bis 25 Gew.%, bezogen auf das Gesamtgewicht der Zusammenstzung, vorhanden ist.

6. Zusammensetzung nach Anspruch 4, dadurch **gekennzeichnet**, dass das Benzoylperoxid in einer Menge von 1 bis 10 Gew.%, bezogen auf das Gesamtgewicht der Zusammensetzung, vorhanden ist.

7. Zusammensetzung nach Anspruch 5, dadurch **gekennzeichnet**, dass das lipophile quartäre Ammoniumsalicylat in einer Menge von 0,1 bis 3 Gew.%, bezogen auf das Gesamtgewicht der Zusammensetzung, vorhanden ist.

8. Zusammensetzung nach einem der Ansprüche 1 bis 7 zur Anwendung als Medikament, dadurch **gekennzeichnet**, dass der geeignete Träger ein pharmazeutisch akzeptabler Träger ist.

9. Zusammensetzung nach einem der Ansprüche 1 bis 7, dadurch **gekennzeichnet**, dass der geeignete Träger ein kosmetisch akzeptabler Träger ist.

10. Zusammensetzung nach einem der Ansprüche 1 bis 9, dadurch **gekennzeichnet**, dass sie in Form von Gelen, Lösungen, Dispersionen, Emulsionen oder Suspensionen vorliegt.

11. Zusammensetzung nach einem der Ansprüche 1 bis 10, dadurch **gekennzeichnet**, dass sie in Form einer Creme, einer Milch, eines Gels, einer Dispersion oder Mikroemulsion, von mehr oder weniger verdickten Zusammensetzungen, eines imprägnierten Tupfers, einer Pommade, eines Stiftes oder in Form einer Seife vorliegt.

12. Zusammensetzung nach einem der Ansprüche 1 bis 11, dadurch **gekennzeichnet**, dass sie zusätzlich zumindest ein anderes Anti-Aknemittel enthält.

13. Zusammensetzung nach einem der Ansprüche 1 bis 12, dadurch **gekennzeichnet**, dass sie zusätzlich zumindest einen Sonnenfilter enthält.

14. Verwendung der Zusamensetzungen nach Anspruch 8 zur Herstellung eines Medikamentes zur Behandlung von Dermatosen.

15. Verwendung der Zusammensetzungen nach Anspruch 14 zur Behandlung von ulzeröser Haut, von Warzen und Dyskeratonie der Haut.

16. Verwendung der Zusammensetzungen nach Anspruch 8 zur Herstellung eines Medikamentes zur Behandlung von Akne.

17. Verfahren zur kosmetischen Behandlung, **gekennzeichnet** durch Auftragen einer Zusammensetzung nach Anspruch 9 auf die Haut, um sie zu reinigen und zu säubern.

## Claims

1. Pharmaceutical or cosmetic composition, characterised in that it is a topical composition containing, in combination in a physiologically acceptable vehicle, benzoyl peroxide and at least one lipophilic quaternary ammonium salicylate corresponding to the general formula (I) :

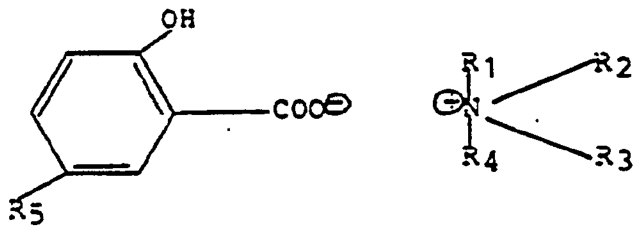 (I)

in which :

(i) $R_1$, $R_2$, $R_3$ and $R_4$, which may be identical or different, denote a saturated linear $C_1$-$C_{18}$ alkyl radical which can bear one or more hydroxyl group(s) at the end of the chain or in the chain ;

(ii) $R_3$ and/or $R_4$ denote(s) a group :

$$R_8 - (OC_2H_3R_7)_n - (OCH_2-CHOH-CH_2)_p$$

in which :

$$0 \leq n \leq 4 \text{ and p denotes 0 or 1 ;}$$

$R_8$ denotes H or a $C_1$ to $C_{18}$ alkyl, alkenyl, alkylcycloalkyl or alkylaryl radical, it being possible for the alkyl groups to be linear or branched and for the aliphatic or aromatic rings optionally to bear one or more $C_1$ to $C_4$ alkyl or alkoxy substiiuent(s) ;

$R_7$ denotes H, $CH_3$ or $CH_2OH$ ;

when

$R_7$ denotes $CH_2OH$, $R_8$ is then other than H and p is equal to 1 ;

in other cases p = 0 ;

the group ($OC_2H_3R_7$) can denote either or both of the following arrangements :

$$-O-CH_2-\underset{R_7}{\underset{|}{CH}}- \; ; \; -O-\underset{R_7}{\underset{|}{CH}}-CH_2-$$

$R_1$ and $R_2$ have the meanings assigned in section (i) ;

(iii) $R_4$ denotes a group :

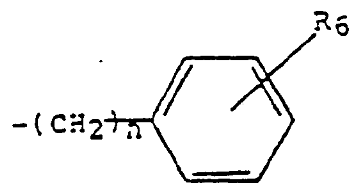

in which n denotes 0 or 1, $R_1$, $R_2$ and $R_3$ having the same meanings as under (i) ; $R_6$ represents a hydrogen, a hydroxyl, a halogen, an alkyl or hydroxyalkyl residue or a $C_1$ to $C_{18}$ acyl residue ;

(iv) $R_1$ and $R_2$ can form an aromatic heterocycle (in which case $R_3$ is non-existent) corresponding to the formula :

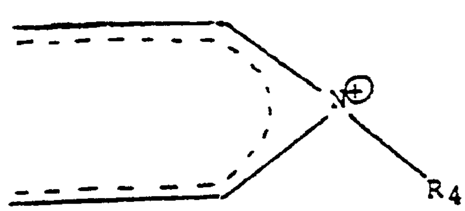

in which $R_4$ has the meanings stated above ; or

(v) $R_1$ and $R_2$ denote a saturated or unsaturated non-aromatic heterocycle optionally interrupted by an oxygen atom ; $R_4$ represents a group defined above and $R_3$ represents a group defined in section (i) ;

(vi) $R_1$, $R_2$ and $R_3$ can form a saturated or unsaturated non-aromatic bicyclic system ; $R_4$ represents a group defined above ; and

$R_5$ denotes a group corresponding to the formula :

$$-\underset{\underset{O}{\|}}{C}-(CH_2)_n-CH_3$$

in which n is an integer vaying between 0 and 16.

2. Composition according to Claim 1, characterised in that, in the formula (I) of the lipophilic quaternary ammonium salicylate, $R_4$ denotes a benzyl group and $R_1$, $R_2$ and $R_3$ have the meanings stated under (i).

3. Composition according to either of Claims 1 and 2, characterised in that the lipophilic quaternary ammonium salicylate is selected from the following compounds : hexadecyltrimethylammonium 5-octanoyl-salicylate, hexadecyltrimethylammonium 5-decanoyl-salicylate, hexadecyltrimethylammonium 5-dodecanoyl-salicylate, hexadecylpyridinium 5-octanoylsalicylate, hexadecylpyridinium 5-decanoylsalicylate, hexadecyl-pyridinium 5-dodecanoylsalicylate, benzyldimethyl-hexadecylammonium 5-octanoylsalicylate, benzyldimethyl-hexadecylammonium 5-decanoylsalicylate, benzyldimethyl-hexadecylammonium 5-dodecanoyl-salicylate, benzyltrimethylammonium 5-decanoylsalicylate, hexadecyldimethyl-hydroxyethylammonium 5-decanoylsalicylate, tetramethyl-ammonium 5-dodecanoylsalicylate, dodecylethyldimethyl-ammonium 5-decanoylsalicylate, trimethyl-β-hydroxyethyl-ammonium 5-decanoylsalicylate, trimethyl-β-hydroxyethylmonium 5-dodecanoylsalicylate, N-dodecyl-N-methyl-morpholinium 5-decanoylsalicylate, N-methyl-N-octyl-piperidinium 5-dodecanoylsalicylate, benzethonium 5-octanoylsalicylate and benzethonium 5-dodecanoyl-salicylate.

4. Composition according to any one of Claims 1 to 3, characterised in that the benzoyl peroxide is present in proportions of 0.1 to 20% by weight relative to the total weight of the composition.

5. Composition according to any one of Claims 1 to 4, characterised in that the lipophilic quaternary ammonium salicylate is present in proportions of 0.01 to 25% by weight relative to the total weight of the composition.

6. Composition according to Claim 4, characterised in that the benzoyl peroxide is present in proportions of 1 to 10% by weight relative to the total weight of the composition.

7. Composition according to Claim 5, characterised in that the lipophilic quaternary ammonium salicylate is present in proportions of 0.1 to 3% by weight relative to the total weight of the composition.

8. Composition according to any one of Claims 1 to 7, for its application as a medicinal product, characterised in that the appropriate vehicle is a pharmaceutically acceptable vehicle.

9. Composition according to any one of Claims 1 to 7, characterised in that the appropriate vehicle is a cosmetically acceptable vehicle.

10. Composition according to any one of Claims 1 to 9, characterised in that it takes the form of gels, solutions, dispersions, emulsions or suspensions.

11. Composition according to any one of Claims 1 to 10, characterised in that it takes the form of a cream, milk, gel, dispersion or microemulsion, of a more or less thickened composition, an impregnated pad, an ointment or a stick, or the form of a cake of soap.

12. Composition according to any one of Claims 1 to 11, characterised in that it comprises, in addition, at least one other anti-acne agent.

13. Composition according to any one of Claims 1 to 12, characterised in that it comprises, in addition, at least one sunscreen agent.

14. Use of the compositions defined according to Claim 8, for the preparation of a medicinal product for the treament of dermatoses.

15. Use of the compositions according to Claim 14, for the treatment of skin ulcers, warts and dyskeratinisations of the skin.

16. Use of the compositions defined according to Claim 8, for the preparation of a medicinal product for the treatment of acne.

17. Cosmetic treament process, characterised in that it consists in applying to the skin a composition as defined in Claim 9, for the purpose of decontaminating or cleansing the skin.